# EUROPEAN PATENT APPLICATION

(11) **EP 2 263 705 A1**
(43) Date of publication of application: **22.12.2010**
(21) Application number: 08790758.0
(22) Date of filing: 30.06.2008
(51) Int. Cl.: A61L 27/00, C12N 5/10, C12N 15/09

(54) **THERAPEUTIC AGENT FOR NERVE INJURY AND METHOD FOR TREATING NERVE INJURY**

(30) Priority: 07.03.2008 JP 2008058447
(71) Applicant: Keio University, Tokyo 108-8345 (JP); Kyoto University, Kyoto-shi Kyoto 606-8501 (JP)
(72) Inventor: OKANO, Hideyuki, Tokyo 160-8582 (JP); NAKAMURA, Masaya, Tokyo 160-8582 (JP); TSUJI, Osahiko, Tokyo 160-8582 (JP); YAMANAKA, Shinya, Kyoto-shi Kyoto 606-8501 (JP); MIURA, Kyoko, Kyoto-shi Kyoto 606-8501 (JP)
(74) Representative: Jones, Helen M.M.
(86) International application number: PCT/JP2008/061845
(87) International publication number: WO 2009/110113

(57) **Abstract**

The present invention provides a therapeutic agent for a nerve injury and a method for treating a nerve injury. One aspect of the invention is the method for treating a nerve injury by administering to a patient with a nerve injury a therapeutic agent for a nerve injury containing a differentiated cell-derived pluripotent cell obtained by forced expression of reprogramming genes such as a combination of the Oct3/4 gene, Sox2 gene, Klf4, and c-myc gene. in a differentiated cell; or cells obtained by inducing the aforementioned differentiated cell-derived pluripotent cells to differentiate into an embryoid body or a neurosphere.

## Description

### [TECHNICAL FIELD]

The present invention relates to a method for treating nerve injuries.

### [BACKGROUND ART]

In recent years, it has become possible to obtain cells having pluripotency similar to embryonic stem cells (hereafter referred to as ES cells) by selecting cells expressing Fbxo15 gene from somatic cells such as fibroblasts in which Oct3/4 gene, Sox2 gene, Klf4 gene, and c-myc gene have been introduced and expressed (Takahashi K. and Yamanaka S. (2006) Cell 126: 663-676; and WO2007/069666). It is considered that if pluripotent stem cells derived from somatic cells obtained as described above are used in regenerative medicine, transplantation of patients' own cells would be possible, thereby minimizing rejectionproblems compared with the cases where embryonic stem cells are used.

While somatic cell-derived pluripotent stem cells (hereafter referred to as induced pluripotent stem cells, or iPS cells) established by using Fbxo15 gene as a marker were closely similar to embryonic stem cells in cell morphology, proliferation ability, and differentiation ability, they were different from ES cells in terms of some characteristics such as gene expression and DNA methylation patterns. Thus, cells were selected by using the expression of the Nanog gene as a marker, and iPS cells having pluripotency more similar to ES cells were established (Okita K et al., (2007) Nature 448: 313-317).

Later, iPS cells were isolated using changes in cell morphology as a marker, instead of the expression of Fbxo15 gene or Nanog gene (Meissner et al. , (2007) Nat Biotechnol 25: 1177-1181). iPS cells were also established by using N-myc instead of c-myc (Blelloch R et al., (2007) Cell Stem Cell 1: 245-247). Further, in mice as well as in humans, iPS cells were established by introducing the three genes of Oct3/4, Sox2 and Klf4, without using c-myc gene (Nakagawa M et al., (2008) Nat Biotechnol 26: 101-106; Wering M et al., (2008) Cell Stem Cell 2: 10-12). In addition, iPS cells were established from hepatocytes and gastric epithelial cells, besides fibroblasts (Aoi T et al., (2008) Science (Published online on February 14, 2008)).

Meanwhile, there has also been a growing body of studies using human cells. Human iPS cells were established by introducing into fibroblasts four genes of Oct3/4, Sox2, Nanog, and lin28 (Yu J et al. , (2007) Science 318: 1917-1920). Human iPS cells were also established by introducing into human fibroblasts and fibroblast-like synoviocytes the Oct3/4 gene, Sox2 gene, Klf4 gene, and c-myc gene--the same combination of genes as used for establishment of mouse iPS cells (Takahashi K et al., (2007) Cell 131: 861-872).

Since iPS cells obtained as described above can be generated using cells derived from the patient to be treated, artificial organs and the like which are free from rejection are expected to be produced by using iPS cells in the field of regenerative medicine.

However, when chimeric mice were generated using Nanog-iPS cells, tumor formation was observed in about 20% of the mice, a value significantly higher than that obtained in similar experiments using ES cells. Such cellular behavior in vivo as well as gene expression and DNA methylation patterns as described above suggest that iPS cells are not the cells having completely the same characteristics as ES cells. It is therefore considered that there is no guarantee that transplantation of iPS cells would result in their differentiation into normal cells.

Therefore, it is still unclear what kind of medical application is possible with the use of iPS cells; nor is it known at all what diseases should be specifically targeted.

### [DISCLOSURE OF THE INVENTION]

### [PROBLEMS TO BE SOLVED BY THE INVENTION]

The problem of the present invention is to identify a target disease for which iPS cells can be applied.

### [MEANS FOR SOLVING THE PROBLEMS]

In one aspect of the present invention, the agent for treating a nerve injury includes a differentiated cell-derived pluripotent stem cell. The differentiated cell-derived pluripotent stem cell mayhave been obtained by forced expression of Oct3/4 gene, Sox2 gene, and Klf4 gene in a differentiated cell; or may have been obtained by forced expression of Oct3/4 gene, Sox2 gene, Klf4 gene, c-myc gene in a differentiated cell. The differentiated cell may be a fibroblast.

In a further aspect of the present invention, the method for treating a nerve injury includes administering a differentiated cell-derived pluripotent stem cell to a patient having the nerve injury. In this method, the differentiated cell-derived pluripotent stem cell may be administered to an injured nerve. The differentiated cell-derived pluripotent stem cell may have been obtained by forced expression of Oct3/4 gene, Sox2 gene, and Klf4 gene in a differentiated cell; or it may have been obtained by forced expression of Oct3/4 gene, Sox2 gene, Klf4 gene, and c-myc gene in a differentiated cell. The differentiated cell may be a fibroblast.

### [BRIEF DESCRIPTION OF THE DRAWINGS]

[FIG. 1] This is a graph showing a time-dependent changes in cell viability in 38C2-Nanog-iPS-SNS-transplantated mice in one embodiment of the present invention.
[FIG. 2] This is a set of photographs showing the result of a histological analysis (hematoxylin and eosin staining) of a transplanted mouse in one embodiment of the present invention.
[FIG. 3] This is a set of photographs showing the result of a histological analysis (DAB staining using HRP-conjugated anti-RFP antibody) of the transplanted mice in one embodiment of the present invention.
[FIG. 4] This is a set of photographs showing the result of a histological analysis (staining with antibodies against cell-type specific markers) of a transplanted mouse in one embodiment of the present invention. As the antibodies against marker s, anti-Hu antibody (for detection of nerve cells) (A), anti-GFAP antibody (for detection of astrocytes) (B), and anti-π-GST antibody (for detection of oligodendrocytes) (C) were used.
[FIG. 5] This isa set of photographs and a graph showing the result of a histological analysis (staining with anti-serotonin receptor antibody) of a transplanted mouse in one embodiment of the present invention.
[FIG. 6] This is a graph showing the result of motor function analysis of a transplanted mouse using the BBB scores in one embodiment of the present invention.

### [DETAILED DESCRIPTION OF THE EMBODIMENTS]

### == Differentiated cell-derived pluripotent stem cells ==

A differentiated cell-derived pluripotent stem cell refers to a cell having pluripotency and self-reproducing ability, which is artificially induced by reprogramming a differentiated cell other than germline cells (such as egg cells, sperm cells and their precursor cells such as oogonia and spermatogonia) or undifferentiated cells derived from embryos at early stages of development (such as embryonic stem cells). The differentiated cells may be derived from an embryo, a fetus, or an adult, and may originate from any animal species, such as mice and humans. The characteristics of the differentiated cell is not particularly limited as long as the cell has at least partly lost intrinsic totipotency of a fertilized cell. Examples of such differentiated cells include fibroblasts, epithelial cells, hepatocytes, etc.

The method for reprogramming is not particularly limited, but it is preferred that cells are induced by introducing nuclear reprogramming factors such that they possess pluripotency and self-reproduction ability. For example, the reprogramming methods as described in WO2005/080598 or WO2007/069666 can be used. These publications are incorporated herein by reference.

The nuclear reprogramming factor is not particularly limited, but preferred is a combination of products of the genes selected from each one member of the Oct gene family, Klf gene family, and Sox gene family. In terms of efficiency of establishment of iPS cells, more preferred is a combination further containing a gene product of the myc gene family. The genes belonging to the Oct gene family include Oct3/4, OctlA, Oct6, etc.; the genes belonging to the Klf gene family include Klf1, Klf2, Klf4, Klf5, etc.; the genes belonging to the Sox gene family include Sox1, Sox2, Sox3, Sox7, Sox15, Sox17, Sox18, etc.; and the genes belonging to the myc gene family include c-myc, N-myc, L-myc, etc. In some cases, gene products of the myc gene family may be substituted with a cytokine such as SCF, bFGF, etc.

Examples of the nuclear reprogramming factors other than the above-described combination include a combination containing Nanog gene and lin-28 gene in addition to a gene from the Oct gene family and a gene from the Sox gene family. It should be noted that when introducing such factors, another type of gene product may be introduced in addition to the genes in the above-described combinations. Examples of such gene products include an immortalization-inducing factor etc.

Since all of the above-mentioned genes are highly conserved among the vertebrates, a gene referred herein includes itshomologues unless the name of a particular animal is indicated. Moreover, mutated genes including polymorphic genes are also encompassed as long as they have a function comparable to that of the wild-type gene product. == The method for preparing differentiated cell-derived pluripotent stem cells ==

To prepare differentiated cell-derived pluripotent cells by using nuclear reprogramming factors, in the case the nuclear reprogramming factor is a protein functioning within a cell, a gene encoding the protein is preferably incorporated into an expression vector, which is introduced into a target differentiated cell, such as a somatic cell, so that the protein is intracellularly expressed (the gene transduction method). The expression vector to be used is not particularly limited, but preferred is a viral vector, and particularly preferred is a retroviral vector or a lentiviral vector. Also, the nuclear reprogramming factor may be introduced into cells by binding a peptide called protein transduction domain (PTD) to the protein, which is added to a culture medium (the protein transduction method). In the case the protein is secreted extracellularly, the factor may be added to a culture medium of differentiated cells during the preparation of the differentiated cell-derived pluripotent cells. If some of the protein factors are expressed within a differentiated cell to be reprogrammed, such proteins do not need to be introduced from the outside.

Cells expressing an undifferentiation marker gene, such as Fbxo15 gene or Nanog gene, are selected in their living state from the differentiated cells into which nuclear reprogramming factors have been introduced. The method for this selection is not particularly limited. For example, a marker gene, such as GFP gene or galactosidase gene, maybe knocked-in into an endogenous Fbxo15 gene or an endogenous Nanog gene and cells expressing the marker gene may be selected. Alternatively, by knocking-in a drug-resistance gene, such as a neomycin resistance gene, a hygromycin tolerance gene, a puromycin resistance gene, etc. into an endogenous Fbxo15 gene or an endogenous Nanog gene, target cells can be easily selected by the drug.

Cells expressing the undifferentiation marker gene are thus selected from the differentiated cells into which the nuclear reprogramming factors have been introduced, and the resulting cell populations are used as the differentiated cell-derived pluripotent cells.

### == The agent for treating a nerve injury ==

The differentiated cell-derived pluripotent cell can be used as a therapeutic agent for a nerve injury. The method for using a therapeutic agent for a nerve injury may be based on a method that has been developed to use embryonic stem cells as a therapeutic agent for a nerve injury, as described in Okada et al., Dev. Biol. vol.275, pp.124-142. 2004.

The therapeutic agent for a nerve injury may contain another component such as a buffer solution containing salt etc. and/or an agent such as antibiotics, in addition to the differentiated cell-derived pluripotent cell. The nervous tissue to be treated is not particularly limited; either the central nervous system (brain or spinal cord) or the peripheral nervous system may be treated. Further, the disease to be treated may be, but not limited to, with any condition (for example, a traumatic disease such as a spinal cord injury; a neurodegenerative disease such as amyotrophic lateralsclerosis, Alzheimer's disease, Parkinson's disease, progressive supranuclear palsy, Huntington disease, multiple system atrophy, and spino-cerebellar degeneration; necrosis of nerve cells resulting from cerebral infarction, intracerebral hemorrhage, etc.), and of any cause (for example, the primary cause being associated with injury, cerebral infarction, etc., and the secondary cause being associated with infection, tumor, etc.), as long as it is a disease or a pathological condition in which nerve cells are damaged.

When the differentiated cell-derived pluripotent cells are administered to a patient, they may be used without a pretreatment. However, to enhance their ability to differentiate into nervous system cells, it is preferred to allow formation of embryoid bodies (EBs) in advance and then administer the EB cells; and it is more preferred to further induce the EBs to differentiate into neural stem cells under culture conditions. For example, by allowing embryoid bodies (EBs) to be formed in the presence of retinoic acid at low concentrations (10⁻⁹ M to 10⁻⁶ M) and then culturing them in a serum-free medium supplemented with FGF-2 (10 to 100 ng/ml), neural stem cells can be differentiated as neurospheres to be administered. Alternatively, EBs may be formed by adding Noggin protein to a culture medium of differentiated cell-derived pluripotent cells. Specifically, a conditioned medium in which Noggin has been transiently expressed by introducing Xenopus Noggin gene into cultured mammalian cells may be added (1 to 50% (v/v)). Recombinant Noggin protein (about 1 µg/ml) may also be used.

The differentiated cell-derived pluripotent cells may be administered either directly or indirectly. For a direct administration, cells may be transplanted to the site of nerve injury, for example. For an indirect administration, cells may be injected intravenously or intraspinally and delivered to the affected site through the circulation of blood and cerebrospinal fluid.

### [EXAMPLES]

### == Cells used ==

In this example, the differentiated cell-derived pluripotent cells were obtained by introducing Oct3/4, Sox2, c-Myc, and Klf4 as nuclear reprogramming factors to mouse embryonic fibroblasts. Specifically, Fbxo15-iPS cells were obtained by a selection using Fbxo15 gene expression as a marker andNanog-iPS cells were obtained by a selection using Nanog gene expression as a marker. In particular, the following clones were used: as the Fbxo15-iPS cells, 4-3 Fbxo15-iPS clone (Takahashi et al., Cell vol.126, pp.663-676, 2006), into which T58A-c-Myc had been introduced, and WT1 Fbxo15-iPS clone (Takahashi et al. , Cell vol.126, pp. 663-676, 2006), into which the wild-type c-Myc had been introduced, were used. As the Nanog-iPS cells, 20D17 Nanog-iPS clone and 38C2 Nanog-iPS clone (Okita et al. , Nature vol. 448, pp. 313-317, 2007), into which T58A-c-Myc had been introduced, as well as 38D2 Nanog-iPS clone (Okita et al., Nature vol.448, pp.313-317, 2007), into which the wild-type c-Myc had been introduced, were used. As controls, mouse embryonic stem cells (Fbxo15 (-/-) RF8 clone) (Tokuzawa et al., Mol. Cell. Biol. vol.23, pp.2699-2708, 2003) andEB3 clone (Niwaet al., Mol. Cell. Biol. vol.22, pp. 1526-1536, 2002) were used.

To enhance the ability of these iPS cells to differentiate into nervous system cells, embryoid bodies (EBs) were allowed to form in the presence of 10⁻⁸ M retinoic acid and then cultured in a serum-free medium supplemented with 20 ng/ml FGF-2 (Okada et al., Dev. Biol. vol.275, pp.124-142, 2004). In seven days in the culture, all of the EBs formed neurospheres (the neurospheres thus obtained are hereafter called iPS-PNS). It was possible to dissociate these iPS-PNS and allow neurospheres to form again under the same conditions repeatedly (these subcultured neurospheres are hereafter called iPS-SNS).

In addition, to obtain clones expressing CBRluc and RFP as the markers for transplanted 38C2-Nanog-iPS-SNS cells, a lentivirus vector harboring an IRES flanked by a red light-emitting click beetle luciferase (CBRluc) gene and a red fluorescent protein (RFP) gene was introduced into the cells. The resulting CBRluc-38C2-Nanog-iPS cells were used for a transplantation experiment into spinal cord-injured mice.

### == Generation of spinal cord-injured mice and cell transplantation ==

Model mice of spinal cord injury were made by inducing traumatic spinal cord injury of the spinal nerve at the 10th thoracic vertebral level, and transplantation of 38C2-Nanog-iPS-SNS was performed, as follows.

First, 8- to 9-week-old C57B16 female mice weighing 20 to 22 g were anesthetized with ketamine (100 mg/kg) and xylazine (10 mg/kg). After laminectomy of the 10th thoracic vertebra, the dorsal surface of the dura mater was exposed, and traumatic spinal cord injury was produced using the Infinite Horizon Impactor (60 kdyn; Precision Systems, Kentucky, IL).

To transplant cells to the injured spinal cord, the injury site was exposed again at 9 days after the injury. Cells of 5 x 10⁵ cells/2µl were introduced into the center of the lesioned area at a rate of 0.5 µl/min using a glass micropipette mounted on a stereotaxic injector (KDS310, Muromachi-kikai, Tokyo, Japan). In this example, the 38C2 clone and EB3 clone were used as Nanog-iPS-SNS and ES-SNS, respectively. Neurospheres of these clones were dissociated and transplanted. As a control, only the culture medium without cells was injected in the same manner as with the cell transplantation.

### == Analysis of spinal cord-injured mice transplanted with 38C2-Nanog-iPS-SNS ==

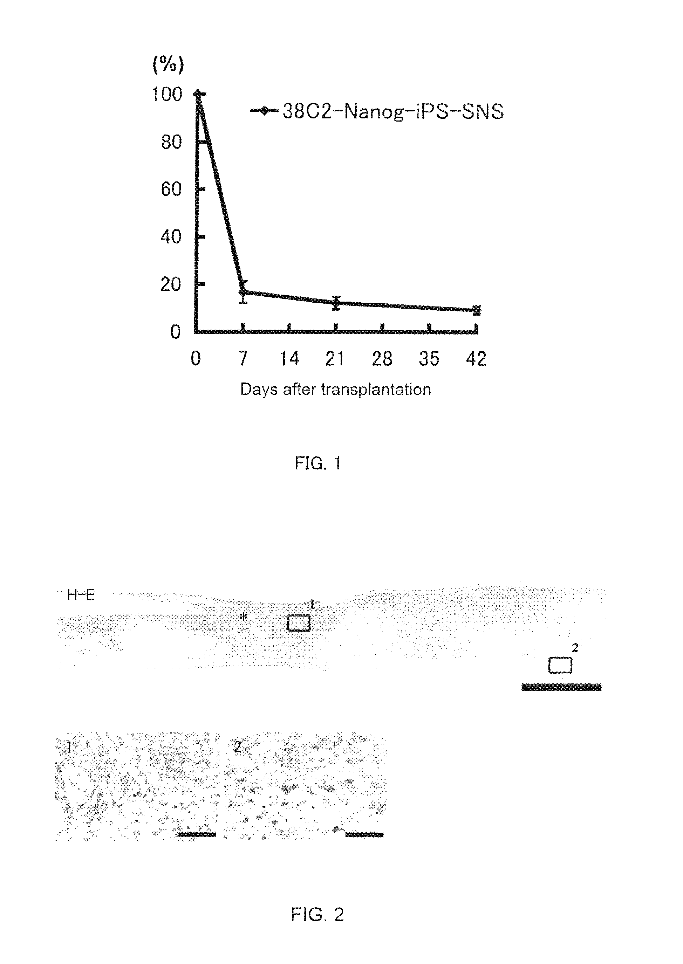

In the bioluminescent imaging (BLI) analysis (Okada et al., Faseb J.vol.19, pp.1839-1841, 2005), the intensity of luminescence by luciferase was measured immediately after transplantation and at days 7, 21, and 35, and was used as an index of the number of cells as follows. D-luciferin (150 mg/kg body weight) was intraperitoneally injected into the mice. For 15 to 40 min after administration of the luciferin, images of the mice with the field-of-view set at 10 cm were repeatedly taken until the highest intensity was obtained. All the images were analyzed using Igor software (WaveMetrics, Lake Oswego, OR) and Living Image software (Xenogen, Alameda, CA). To quantify the number of photons, a fixed transplantation area was defined and analyzed in each of the mice. Using the number of photons obtained at each time point, ratios to the initial value were calculated and plotted in FIG. 1. As shown in FIG. 1, approximately 60% of the transplanted cells were lost by day 7 after transplantation, then the signals of the transplanted cells were gradually reduced, and at day 35 about 18% of the transplanted cells were found to be alive.

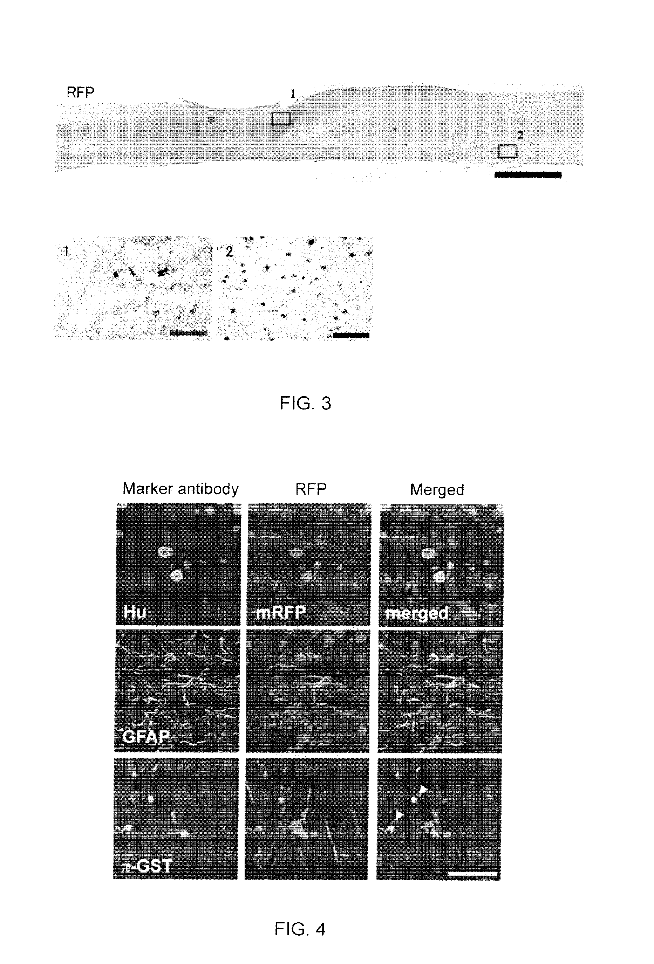

At six weeks after the traumatic injury, histological analysis of the 38C2-Nanog-iPS-SNS cell-transplanted mice was performed. In the panel H-E of FIG. 2 (hematoxylin and eosin staining) and in the panel RFP of FIG. 3 (DBA staining using HRP-conjugated anti-RFP antibody), an asterisk indicates the cell transplantation site, and magnified images of the areas indicated by square 1 (the periphery of the nerve injury area) and square 2 (the white matter in the anterior of the transplantation site), respectively, are shown at the bottom. As a result, no sign of tumorigenesis was observed (FIG. 2). While most of the transplanted cells were observed around the nerve injury area (FIG. 3-1), there were also some cells that had moved toward the posterior by approximately 4 mm (FIG. 3-2).

Sections of the injured spinal cord were stained using antibodies against cell-type specific markers. The 38C2-derived transplanted cells detected with RFP were round to have differentiated into three cell species: nerve cells (FIG. 4A; the marker was Hu), astrocytes (FIG. 4B; the marker was GFAP), and oligodendrocytes (FIG. 4C; the marker was π-GST) (FIG. 4).

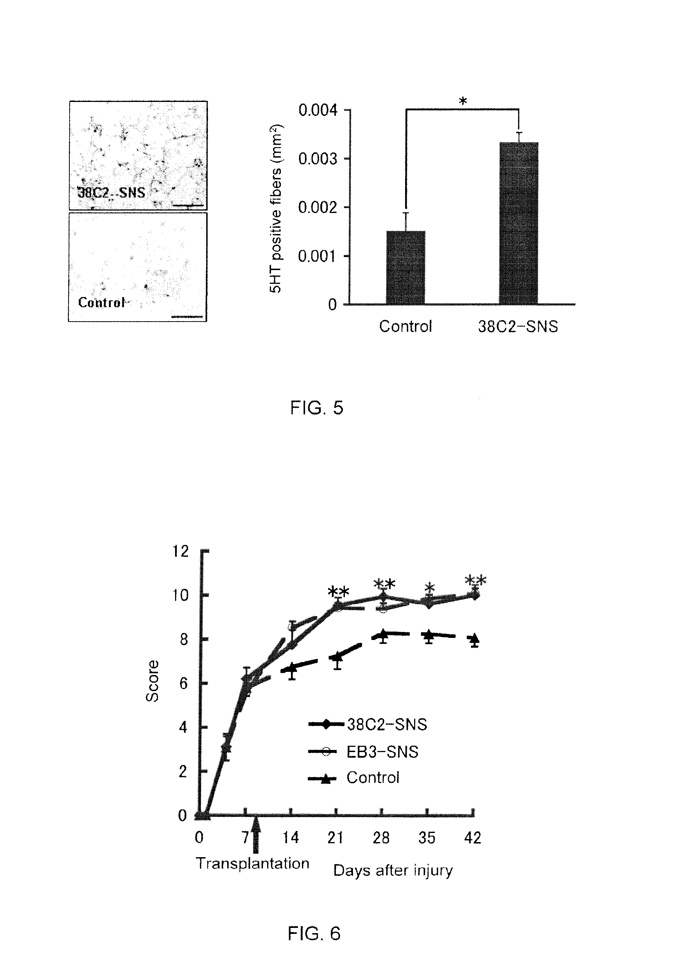

Further, to examine the number of serotonergic neurones, staining was performed using anti-serotonin receptor antibody. The images obtained by microscopic observation as well as the measured areas of the stained portions to quantify the numbers of neurons are shown in FIG. 5. There were significant reduction of the number of the 5HT-positive serotonergic neurons in both the microscope images and the stained areas when the culture medium without cells (Control), which served as the control, was injected as compared with the number of the neurons when 38C2-iPS-SNS (38C2-SNS) were transplanted (FIG. 5).

Next, the motor function of hindlimbs was evaluated by the Basso-Beattie-Bresnahan (BBB) score (Basso et al., J. Neurotrauma vol.12, pp.1-21, 1995) at every seven days till day 42. In the motor function analysis, comparison was made among the three groups: 38C2-Nanog-iPS-SNS-transplanetd group (n=20), EB3-ES-SNS-transplanted group (n=15), and the group receiving only the cell-free medium (n=12). In all three groups, mice were completely paralyzed immediately after the induction of spinal cord injury, but they all gradually recovered. However, at 6 weeks after the operation, the mice in the medium-injected group were unable to support their weight on the hindlimbs, whereas the mice in the 38C2-Nanog-iPS-SNS-transplanted group recovered enough to be able to lift their trunks. As the result of the comparison of the BBB scores, almost the same degree of recovery was observed in the 38C2-Nanog-iPS-SNS group (10.03+/-0.47) and EB3-ES-SNS group (10.10+/-0.24) at 6th postoperative week, with a significant difference from the recovery in the group receiving only the cell-free medium (8.08+/-0.39) (FIG. 6). Also from the clinical observations, the 38C2-Nanog-iPS-SNS-transplanted mice exhibited marked recovery of weight-supported plantar stepping.

In conclusion, by transplanting iPS cells to nerve injured mice, the nerve injury can be treated.

### [INDUSTRIAL APPLICABILITY]

The present invention has made it possible to provide a therapeutic agent for a nerve injury containing iPS cells as well as a method for treating a nerve injury using iPS cells.

## Claims

1. An agent for treating a nerve injury, comprising a differentiated cell-derived pluripotent stem cell.

2. The agent of claim 1, wherein the differentiated cell-derived pluripotent stem cell has been obtained by forced expression of Oct3/4 gene, Sox2 gene, and Klf4 gene in the differentiated cell.

3. The agent of claim 1, wherein the differentiated cell-derived pluripotent stem cell has been obtained by forced expression of Oct3/4 gene, Sox2 gene, Klf4 gene, and c-myc gene in the differentiated cell.

4. The agent of claim 1, wherein the differentiated cell is a fibroblast.

5. A method for treating a nerve injury, comprising administering a differentiated cell-derived pluripotent stem cell to a patient having the nerve injury.

6. The method of claim 5, wherein the differentiated cell-derived pluripotent stem cell is transplanted to an injured nerve.

7. The method of claim 5, wherein the differentiated cell-derived pluripotent stem cell has been obtained by forced expression of Oct3/4 gene, Sox2 gene, and Klf4 gene in the differentiated cell.

8. The method of claim 5, wherein the differentiated cell-derived pluripotent stem cell has been obtained by forced expression of Oct3/4 gene, Sox2 gene, Klf4 gene, and c-myc gene in the differentiated cell.

9. The method of claim 5, wherein the differentiated cell is a fibroblast.
